# EUROPEAN PATENT SPECIFICATION

(11) **EP 1 246 815 B1**
(45) Date of publication and mention of the grant of the patent: **17.03.2004**
(21) Application number: 00984923.3
(22) Date of filing: 20.12.2000
(51) Int. Cl.: C07D 403/02, C07D 403/14, C07D 413/14, C07D 405/14, A61K 31/496, A61K 31/5377, A61K 31/55, A61P 25/18, A61P 25/22, A61P 25/24

(54) **PHENYLPIPERAZINYL DERIVATIVES**
PHENYLPIPERAZINYL-DERIVATE
DERIVES DE PHENYLPIPERAZINYLE

(30) Priority: 30.12.1999 DK 188699
(43) Date of publication of application: 09.10.2002
(73) Proprietor: H. Lundbeck A/S, 2500 Valby-Copenhagen (DK)
(72) Inventor: RUHLAND, Thomas, DK-2500 Valby (DK); KEHLER, Jan, DK-2800 Kgs. Lyngby (DK); ANDERSEN, Kim, DK-2830 Virum (DK); BANG-ANDERSEN, Benny, DK-2200 Copenhagen N (DK); ROTTLÄNDER, Mario, DK-2500 Valby (DK)
(74) Representative: Meidahl Petersen, John
(86) International application number: PCT/DK2000/000720
(87) International publication number: WO 2001/049677

(56) References cited:
- EP-A1- 0 376 607
- WO-A1-94/21630
- WO-A1-94/22839
- WO-A1-94/24105
- WO-A1-95/29911
- WO-A1-96/04250
- WO-A1-97/26252

## Description

The present invention relates to a novel class of substituted phenylpiperazinyl,-piperidinyl and -tetrahydropyridinyl derivatives having affinity for dopamine D4 receptors. The compounds are useful in the treatment of certain psychiatric and neurologic disorders, including psychosis.

### Background of the Invention.

Dopamine D₄ receptors belong to the dopamine D₂ subfamily of receptors, which is considered to be responsible for the antipsychotic effects of neuroleptics. The side effects of neuroleptic drugs, which primarily exert their effect via antagonism of D₂ receptors, are known to be due to D₂ receptor antagonism in the striatal regions of the brain. However, dopamine D₄ receptors are primarily located in areas of the brain other than striatum, suggesting that antagonists of the dopamine D₄ receptor will be devoid of extrapyramidal side effects. This is illustrated by the antipsychotic clozapine, which exerts higher affinity for D₄ than D₂ receptors, and is lacking extrapyramidal side effects (Van Tol et aI. *Nature* **1991**, *350*, 610; Hadley *Medicinal Research Reviews* **1996**, *16*, 507-526 and Sanner *Exp. Opin. Ther. Patents* **1998,** *8*, 383-393).

A number of D₄ ligands, which were postulated to be selective D₄ receptor antagonists (L-745,879 and U-101958) have been shown to posses antipsychotic potential (Mansbach et al. *Psychopharmacology* **1998**, *135*, 194-200). However, recently is has been reported that these compounds are partial D₄ receptor agonists in various *in vitro* efficacy assays (Gazi et al. *Br. J. Pharmacol.* **1998**, *124*, 889-896 and Gazi et al. *Br. J. Pharmacol*. **1999,** *128*, 613-620). Furthermore, it was shown that clozapine, which is an effective antipsychotic, is a silent antagonist (Gazi et al. *Br. J. Pharmacol*. **1999**, *128*, 613-620).

Consequently, D₄ ligands, which are partial D₄ receptor agonists or antagonists, may have beneficial effects against psychoses.

Dopamine D₄ antagonists may also be useful for the treatment of cognitive deficits (Jentsch et al. *Psychopharmacology* **1999,***142, 78-84*.

It has also been suggested that dopamine D₄ antagonists may be useful to reduce dyskinesia occurring as a result of the treatment of Parkinson's disease with L-dopa (Tahar et al. *Eur. J. Pharmacol*. **2000,** *399*, 183-186).

Dopamine D₃ receptors also belong to the dopamine D₂ subfamily of receptors, and they are preferentially located in limbic regions of the brain (Sokoloff et al. *Nature*, **1990,** *347*, 146-151), such as the nucleus accumbens, where dopamine receptor blockade has been associated with antipsychotic activity (Willner *Int. Clinical Psychopharmacology* **1997,** *12*, 297-308). Furthermore, an elevation of the level of D₃ receptors in the limbic part of schizophrenic brains has been reported (Gurevich et al. *Arch. Gen. Psychiatry* **1997,** *54*, 225-32). Therefore, D₃ receptor antagonists may offer the potential for an effective antipsychotic therapy, free of the extrapyramidal side effects of the classical antipsychotic drugs, which primarily exert their effect by blockade of D₂ receptors (Shafer et al. *Psychopharmacology* **1998,** *135,* 1-16; Schwartz et al. *Brain Research Reviews* **2000**, *31*, 277-287).

Moreover, D₃ receptor blockade results in a slight stimulation in the prefrontal cortex (Merchant et al. *Cerebral Cortex* **1996**, *6*, 561-570), which could be beneficial against negative symptoms and cognitive deficits associated with schizophrenia. In addition, dopamine D₃ antagonists can reverse D₂ antagonist-induced EPS (Millan et al. *Eur. J. Pharmacol*. **1997**, *321*, R7-R9) and do not cause changes in prolactin (Reavill et al. *J. Pharmacol. Exp. Ther*. **2000,** *294*, 1154-1165). Consequently, D₃ antagonistic properties of an antipsychotic drug could reduce the negative symptoms and cognitive deficits and result in an improved side effect profile with respect to EPS and hormonal changes.

Dopamine D₃ agonists have also been considered relevant in the treatment of schizophrenia (Wustow et al. *Current Pharmaceutical Design* **1997**, *3*, 391-404).

A number of dopamine D₄ ligands, which can be described by the general formula 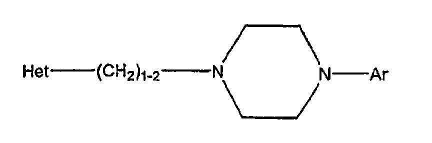 wherein Het is 3-pyrrolo[2,3-b]pyridinyl, 2-benzimidazolyl, 3-indazolyl, 2-indolyl, 3-benzofuranyl, imidazo[1,2-a]pyridinyl, 3-furo[2,3-b]pyridinyl and 3-benzofuranyl and Ar is optionally substituted phenyl, have been described in WO 9420497, WO 9422839, WO 9421630, WO 9424104, WO 9909025, WO 9529911, WO 9625414, US 5700802 and *J. Med. Chem*. **1996**, *39(19)*, 1941-2.

WO 9604250 relates to certain 1-(3-piperazinopropyl or 3-piperidinopropyl)benzimidazoles having dopaminergic activity.

According to the present invention a novel class of dopamine D₄ ligands is provided.

Many of the compounds of the invention also have affinity for the dopamine D₃ receptor and as mentioned above, dopamine D₃ antagonistic properties of an antipsychotic drug may reduce the negative symptoms and cognitive deficits of schizophrenia and result in an improved side effect profile.

Some of the compounds of the invention also have affinity for 5-HT receptors such as the 5-HT_{1A} and the 5-HT_{2A} receptor and/or the 5-HT transporter.

### Summary of the Invention

Accordingly, the present invention relates to novel compounds having the formula I 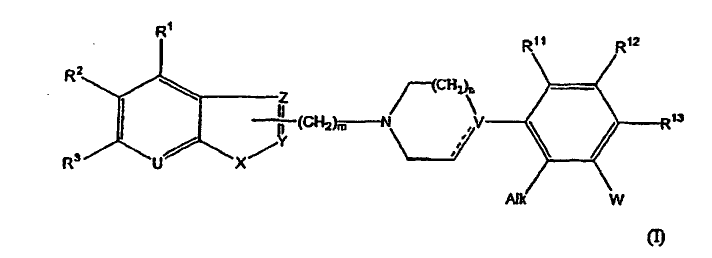 wherein X is N, NR, CR CHR, O or S, Z and Y are selected from N C and CR, and R is hydrogen, C₁₋₆-alkyl or acyl; provided that at least one of X, Y and Z is a heteroatom;
U is CR⁴ or N;
V is C, CH or N, and the dotted line emanating from X indicates a bond when X is C and no bond when X is N or CH;
Alk is C₁₋₆-alkyl;
W is a ring of formula -NR¹⁴R¹⁵ wherein R¹⁴ and R¹⁵ together with the nitrogen atom to which they are attached form a 5 to 6 membered ring, which may contain an additional heteroatom selected from N, O and S, and which may be substituted one or more times with substituents selected from halogen, trifluoromethyl, nitro, cyano, C₁₋ ₆-alkyl, C₂₋₆-alkenyl, C₂₋₆-alkynyl, C₁₋₆-alkoxy, C₁₋₆-alkylthio, C₁₋₆-alkylsulfonyl, hydroxy, hydroxy-C₁₋₆ alkyl, amino, C₁₋₆-alkylamino, di-(C₁₋₆-alkyl)amino, acyl, aminocarbonyl, C₁₋₆-alkylaminocarbonyl, and a di-(C₁₋₆-alkyl)aminocarbonyl; or W is a phenoxy or phenylsulfanyl group, which may be substituted one or more times with substituents selected from halogen, trifluoromethyl, nitro, cyano, C₁₋₆-alkyl, C₂₋₆-alkenyl, C₂₋₆-alkynyl, C₁₋₆-alkoxy, C₁₋₆-alkylthio, C₁₋₆-alkylsulfonyl, hydroxy, hydroxy-C₁₋₆ alkyl, amino, C₁₋₆-alkylamino, di-(C₁₋₆-alkyl)amino, acyl, aminocarbonyl, C₁₋₆-alkylaminocarbonyl, di-(C₁₋₆-alkyl)aminocarbonyl, a ethylenedioxy and a methylene dioxy group;
R¹, R², R³, R⁴, R¹¹, R¹² and R¹³ are selected from hydrogen, halogen, trifluoromethyl, nitro, cyano, C₁₋₆-alkyl, C₂₋₆-alkenyl, C₂₋₆-alkynyl, C₁₋₆-alkoxy, C₁₋₆-alkylthio, C₁₋₆-alkylsulfonyl, hydroxy, hydroxy-C₁₋₆ alkyl, amino, C₁₋₆-alkylamino, di-(C₁₋₆-alkyl)amino, acyl, aminocarbonyl, C₁₋₆-alkylaminocarbonyl and di-(C₁₋₆-alkyl)aminocarbonyl,
n is 1, or 2; m is 1, 2, 3, 4, 5 or 6;
and acid addition salts thereof.

In one embodiment of the invention W is an optionally substituted morpholino, piperidinyl or pyrrolidinyl group, in particular an optionally substituted morpholino group.

In another embodiment of the invention, W is an optionally substituted phenoxy or phenylsulfanyl group.

In a particular embodiment of the invention, Alk is methyl.

In another particular embodiment of the invention U is CR⁴, X is NR, Y is CH, Z is C and the thus formed indole is attached to the remainder of the molecule via position 3.

According to a further embodiment, the present invention relates to compounds of formula I wherein m is 3, 4, 5 or 6..

The compounds of the invention have been found to show affinity for the dopamine D₄ receptor and many of the compounds also have alfinity for the dopamine D₃ receptor. The compounds of the invention are therefore considered useful for the treatment of psychoses including the positive and negative symptoms of schizophrenia.

Further, many of the compounds have been found also to show affinity for serotonergic receptors and /or to have 5-HT reuptake inhibiting effect.

Such compounds may be useful for the treatment of disorders caused by imbalances in the serotonergic system including affective disorders, such as generalised anxiety disorder, panic disorder, obsessive compulsive disorder, depression and aggression.

Compounds with combined effects at dopamine D₄ and 5-HT receptors and/or the 5-HT transporter may have the further benefit of improved effect on other psychiatric symptoms which are seen in schizophrenic patients such as depressive and anxiety symptoms.

In another aspect, the present invention provides a pharmaceutical composition comprising at least one compound of Formula I as defined above or a pharmaceutically acceptable acid addition salt thereof in combination with one or more pharmaceutically acceptable carriers or diluents.

In a further aspect, the present invention provides the use of a compound of Formula I as defined above or an acid addition salt thereof for the manufacture of a pharmaceutical preparation for the treatment of the above mentioned disorders.

### Detailed Description of the Invention

Some of the compounds of general Formula I exist as optical isomers thereof and such optical isomers are also embraced by the invention.

The term C₁₋₆ alkyl refers to a branched or unbranched alkyl group having from one to six carbon atoms inclusive, such as methyl, ethyl, 1-propyl, 2-propyl, 1-butyl, 2-butyl, 2-methyl-2-propyl and 2-methyl-1-propyl.

Similarly, C₂₋₆ alkenyl and C₂₋₆ alkynyl, respectively, designate such groups having from two to six carbon atoms, including one double bond and triple bond respectively, such as ethenyl, propenyl, butenyl, ethynyl, propynyl, and butynyl.

As used herein the term acyl refers to a formyl or C₁₋₆ alkylcarbonyl.

The terms C₁₋₆ alkoxy, C₁₋₆ alkylthio, C₁₋₆ alkylsulfonyl, C₁₋₆ alkylamino, di-(C₁₋₆-alkyl)amino, C₁₋₆-alkylaminocarbonyl, di-(C₁₋₆-alkyl)aminocarbonyl, hydroxy-C₁₋₆ alkyl, C₁₋₆ alkylcarbonyl etc. designate such groups in which the C₁₋₆ alkyl group is as defined above.

Halogen means fluoro, chloro, bromo or iodo.

The ring of formula -NR¹⁴R¹⁵ wherein R¹⁴ and R¹⁵ together with the nitrogen atom to which they are attached form a 5 to 6 membered ring of carbon atoms, which may contain an additional heteroatom selected form N, O and S, is preferably a saturated ring.

Preferred compounds of the invention are compounds selected from:
1-(2-methyl-3-[3-(dimethylamino)phenoxy]phenyl)-4-[3-(1*H*-indol-3-yl)-propyl]piperazine,
1-[3-(1*H*-Indol-3-yl)propyl]-4-[2-methyl-3-(morpholin-4-yl)-phenyl]piperazine,
1-[2-methyl-3-(morpholin-4-yl)phenyl]-4-[4-(1*H*-indol-3-yl)butyl]piperazine,
1-[2-methyl-3-(morpholin-4-yl)phenyl]-4-[3-(5-fluoro-1*H*-indol-3-yl)propyl]piperazine,
1-[2-methyl-3-(morpholin-4-yl)phenyl]-4-[4-(5-fluoro-1*H*-indol-3-yl)butyl]piperazine,
1-[3-(5-chloro-1*H*-Indol-3-yl)propyl]-4-[2-methyl-3-(morpholin-4-yl)-phenyl]piperazine,
1-{3-[3-(Diethylamino)phenoxy]-2-methylphenyl}-4-[3-(5-fluoro-1*H*-indol-3-yl)propyl]piperazine,
1-[2-Methyl-3-(3,4,5-trimethoxyphenoxy)phenyl]-4-[3-(5-fluoro-1*H*-indol-3-yl)propyl]piperazine,
1-{3-[3-(Diethylamino)phenoxy]-2-methylphenyl}-4-[3-(5-chloro-1*H*-indol-3-yl)propyl]piperazine,
1-{3-[3-(Diethylamino)phenoxy]-2-methylphenyl}-4-[3-(5,7-difluoro-1*H*-indol-3-yl)propyl]piperazine,
1-[2-Methyl-3-(3,4,5-trimethoxyphenoxy)phenyl}-4-[3-(5-chloro-1*H*-indol-3-yl)propyl]piperazine,
1-{3-[3-(Diethylamino)phenoxy]-2-methylphenyl}-4-[3-(1*H*-indol-3-yl)propyl]piperazine,
1-[3-(2,6-Dimethylmorpholin-4-yl)-2-methylphenyl]-4-[3-(5-fluoro-1*H*-indol-3-yl)propyl]-1,4-diazepan,
1-{3-[3-(Dimethylamino)phenoxy]-2-methylphenyl}-4-[3-(5-fluoro-1*H*-indol-3-yl)propyl]piperazine,
1-[3-(1,3-Benzodioxolan-5-yloxy)-2-methylphenyl]-4-[3-(1*H*-indol-3-yl)propyl]piperazine,
1-[3-(2,6-Dimethylmorpholin-4-yl)-2-methylphenyl]-4-[3-(5-fluoro-1*H*-indol-3-yl)propyl]piperazine,
1-{3-[3-(Diethylamino)phenoxy]-2-methylphenyl}-4-[4-(5-fluoro-1*H*-indol-3-yl)butyl]piperazine,
1-[3-(Morpholin-4-yl)-2-methylphenyl]-4-[3-(5-chloro-1*H*-indol-3-yl)propyl]piperazine,
1-{3-[3-(Dimethylamino)phenoxy]-2-methylphenyl}-4-[3-(5-chloro-1*H*-indol-3-yl)propyl]piperazine,
(S)-1-[3-(2-Hydroxymethylpyrrolidin-1-yl)-2-methylphenyl]-4-[3-(1*H*-indol-3-yl)propyl]piperazine,
(S)-1-[3-(2-Hydroxymethylpyrrolidin-1-yl)-2-methylphenyl]-4-[3-(5-fluoro-1*H*-indol-3-yl)propyl]piperazine,
1-[3-(1,3-Benzodioxolan-5-yloxy)-2-methylphenyl]-4-[3-(5-fluoro-1*H*-indol-3-yl)propyl]piperazine,
1-{3-[3-(Dimethylamino)phenoxy]-2-methylphenyl}-4-[4-(5,7-difluoro-1*H*-indol-3-yl)butyl]piperazine,
(S)-1-[3-(2-Hydroxymethylpyrrolidin-1-yl)-2-methylphenyl]-4-[3-(5,7-difluoro-1*H*-indol-3-yl)propyl]piperazine,
1-[3-(1,3-Benzodioxolan-5-yloxy)-2-methylphenyl]-4-[3-(5,7-difluoro-1*H*-indol-3-yl)pxopyl]piperazine,
1-[3-(2,6-Dimethylmorpholin-4-yl)-2-methylphenyl]-4-[3-(1*H*-indol-3-yl)propyl]-1,4-diazepane,
1-[3-(2,6-Dimethylmorpholin-4-yl)-2-methylphenyl]-4-[3-(5,7-difluoro-1*H*-indol-3-yl)propyl]piperazine,
1-{3-[3-(Diethylamino)phenoxy]-2-methylphenyl}-4-[3-(5,7-difluoro-1*H*-indol-3-yl)propyl]-1,4-diazepane,
1-{3-[3-(Dimethylamino)phenoxy]-2-methylphenyl}-4-[3-(1*H*-indol-3-yl)propyl]]-1,4-diazepane,
1-{3-[3-(Dimethylamino)phenoxy]-2-methylphenyl}-4-[3-(5,7-difluoro-1*H*-indol-3-yl)propyl]piperazine,
1-[3-(Morpholin-4-yl)-2-methylphenyl]-4-[3-(5-fluoro-1*H*-indol-3-yl)propyl]-1,4-diazepane,
1-{3-[3-(Diethylamino)phenoxy]-2-methylphenyl}-4-[4-(5-chloro-1*H*-indol-3-yl)butyl]piperazine,
1-[3-(1,3-Benzodioxolan-5-yloxy)-2-methylphenyl]-4-[3-(5-fluoro-1*H*-indol-3-yl)propyl]-1,4-diazepane,
1-{3-[3-(Diethylamino)phenoxy]-2-methylphenyl}-4-[4-(5,7-difluoro-1*H*-indol-3-yl)butyl]piperazine,
1-{3-[3-(Morpholin-4-yl)phenoxy]-2-methylphenyl}-4-[3-(5-fluoro-1*H*-indol-3-yl)propyl]-1,4-diazepane,
(S)-1-[3-(2-Hydroxymethylpyrrolidin-1-yl)-2-methylphenyl]-4-[3-(5-chloro-1*H*-indol-3-yl)propyl]piperazine,
1-[3-(1,3-Benzodioxolan-5-yloxy)-2-methylphenyl]-4-[3-(5-chloro-1*H*-indol-3-yl)propyl]-1,4-diazepane, and
1-[3-(2,6-Dimethylmorpholin-4-yl)-2-methylphenyl]-4-[3-(5,7-difluoro-1*H*-indol-3-yl)propyl]-1,4-diazepane
and pharmaceutically acceptable acid addition salts thereof.

The acid addition salts of the compounds of the invention may be pharmaceutically acceptable salts formed with non-toxic acids. Exemplary of such organic salts are those with maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic, and theophylline acetic acids, as well as the 8-halotheophyllines, for example 8-bromotheophylline. Exemplary of such inorganic salts are those with hydrochloric, hydrobromic, sulfuric, sulfamic, phosphoric and nitric acids.

The compounds of the invention may be prepared as follows:
a) Reducing the carbonyl group of a compound of formula II 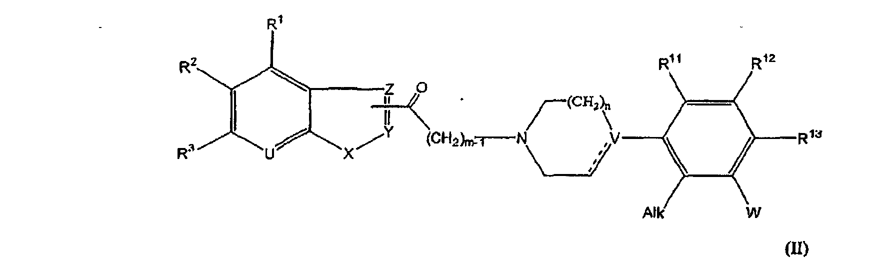 wherein R¹-R³, R¹¹-R¹³, Alk, U, V, X, Y, Z, W, n, m and the dotted line are as previously defined,
b) alkylating an amine of formula III with a reagent of formula IV 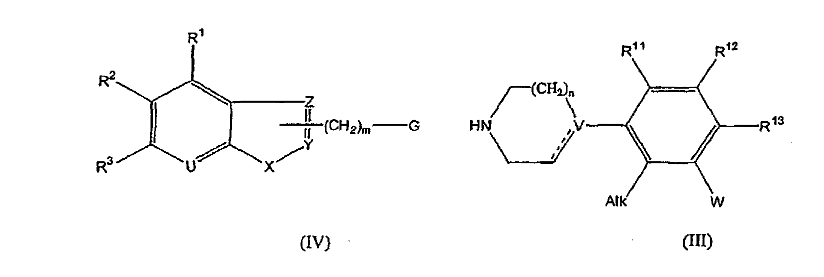 wherein R¹-R³, R¹¹-R¹³, alk, U, V, X, Y, Z, W, n, m and the dotted line are as previously defined, and G is a suitable leaving group such as halogen, mesylate or tosylate;
c) reductive alkylation of an amine of the formula III with a reagent of formula V 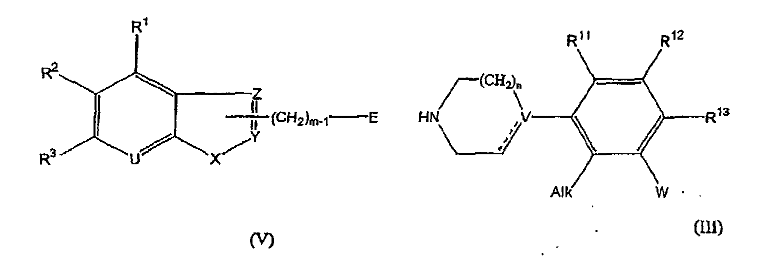 wherein R¹-R³, R¹¹-R¹³, alk, U, V, X, Y, Z, W, n, m and the dotted line are as previously defined, and E is either an aldehyde group or an activated carboxylic acid group;
d) reducing the amide group of a compound of formula VI 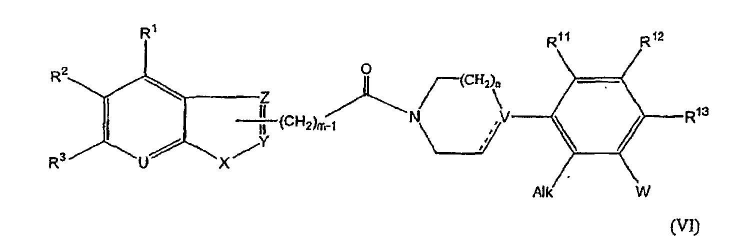 wherein R¹-R³, R¹¹-R¹³, alk, U, V, X, Y, Z, W, n, m and the dotted line are as previously defined.
e) reacting a compound of formula 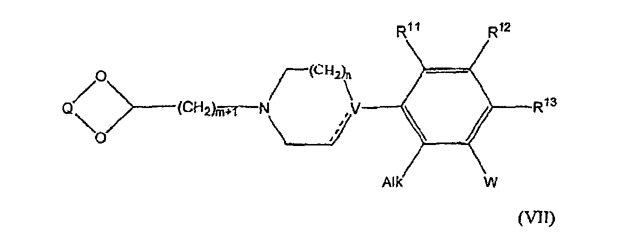
wherein R¹¹-R¹³, alk, W, V, n, m and the dotted line are as previously defined and Q(OH)₂ is optionally substituted ethylene glycol or propylene glycol, or a polymer bound diol; with a compound of the formula 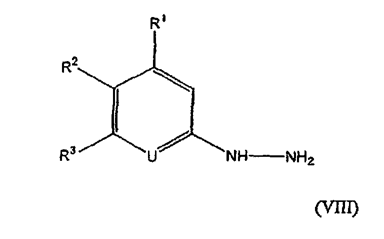 wherein R¹ - R³ and U is as defined above.

The reduction according to method a) is preferably carried out in an inert organic solvent such as diethyl ether or tetrahydrofuran in the presence of alane or lithium aluminium hydride from 0 °C to reflux temperature. Starting compounds of formula (II) are generally prepared by alkylating an amine of formula III with an appropriately substituted (indol-3-yl)-1-oxoalkylhalide. Appropriately substituted (indol-3-yl)-1-oxoalkylhalides can be synthesised by methods described in the literature, e.g. Bergman, J. et al. *Tetrahedron* **1990,** *46*, 6061or in standard works such as Houben-Weyl, Methoden der organischen Chemie (Methods of Organic Chemistry), Georg-Thieme-Verlag, Stuttgart; Organic Reactions, John Wiley & Sons, Inc. New York, namely under reaction conditions such as those which are known and suitable for such reactions

The alkylation according to method b) is conveniently performed in an inert organic solvent such as a suitably boiling alcohol or ketone, preferably in the presence of an organic or inorganic base (potassium carbonate, diisopropylethylamine or triethylamine). Alternatively, the alkylation can be performed at a fixed temperature, which is different from the boiling point, in one of the above mentioned solvents or in dimethyl formamide (DMF), dimethylsulfoxide (DMSO), or *N*-methylpyrrolidin-2-one (NMP), preferably in the presence of a base.

Key intermediates such as amines of the formula III can be prepared by sequential substitution of a suitable η⁶-1,3-dichloro-2-alkylphenyl-η⁵-cyclopentadienyliron(II) hexafluorophosphate by the desired nucleophiles followed by decomplexation of the iron-ion and removal of protecting groups as described in the examples and in the reaction scheme below: 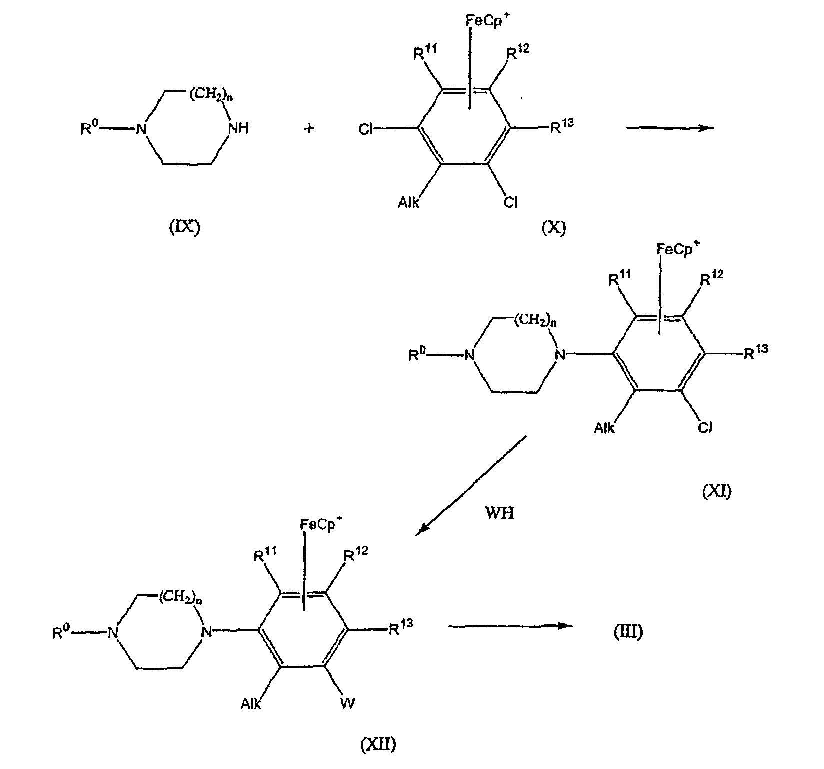 wherein R¹¹ - R¹³, n, alk and W are as defined above, Cp is cyclopentadienyl and R⁰ is an appropriate protecting group such as an ethoxy-, methoxy- or 2-methyl-2-propyloxy-carbonyl group or a benzyl group, or a suitable solid support such as a Merrifield resin or a solid supported carbamate group such as the wang resin based carbamate linker (Zaragoza *Tetrahedron Lett*. **1995,** *36*, 8677-8678). η⁶-1,3-dichloro-2-alkylphenyl-η⁵-cyclopentadienyliron(II) hexafluorophosphate of formula (X) can be prepared from commercially available ferrocene and 1,3-dichloro-2-alkylbenzene analogously to methods described in the literature e.g. Pearson et al. *J. Org. Chem*. **1994,** *59*, 4561. The mono substituted cyclic diamines of formula (IX) are prepared from commercially available starting materials by methods obvious to the chemist skilled in the art. The mono substituted cyclic diamine of formula (IX) are reacted with η⁶-1,3-dichlorotoluene-η⁵-cyclopentendienyliron(II)hexafluorophosphate at elevated temperature in an aprotic solvent such as dry tetrahydrofuran using an appropriate base such as potassium carbonate. The thus formed mono chloro derivatives of formula (XI) are subsequently reacted with a nuclephile of WH, with W as previously defined, in an aprotic solvent such as dry tetrahydrofuran either by the use of an appropriate base such as potassium carbonate or by deprotonation of the nucleophile of formula WH using a base such as sodium hydride prior to the reaction. Decomplexation, performed according to literature procedures (Pearson et al. *J. Org. Chem*. **1996,** *61*, 1297-1305), followed by deprotection by methods obvious to the chemist skilled in the art or cleavage from the solid support according to literature procedures (Zaragoza *Tetrahedron Lett*. **1995,** *36*, 8677-8678 and Conti et al. *Tetrahedron Lett*. **1997**, *38* 2915-2918) afforded the desired secondary amines of formula III.

Nucleophiles of formula WH are either commercially available, prepared by methods obvious to the chemist skilled in the art or according to literature procedures (Guillaumet and Hretani *J*. *Heterocyclic Chem*. **1989,** *26*, 193-196, 1989).

The intermediates of formula IV are either commercially available, prepared by methods obvious to the chemist skilled in the art or according to literature procedures. Haloalkylindoles of formula IV capable of alkylating can be prepared by literature methods (Crooks et al. *J. Med*. Chem. **1983,** *26*, 1470, or analogues to the method described by e.g. Brodfuehrer et al. *J. Org. Chem*. **1997**, *62*, 9192 and Anelli, et al. *J. Org. Chem*. **1987,** *52*, 2559)

The reductive alkylation according to method c) is performed by standard literature methods. The reaction can be performed in two steps, i.e. coupling of derivatives of formula III and the reagent of formula V by standard methods via the carboxylic acid chloride or by use of coupling reagents such as e.g. dicyclohexyl carbodiimide followed by reduction of the resulting amide with lithium aluminium hydride or alane. The reaction can also be performed by a standard one-pot procedure. Carboxylic acids or aldehydes of formula V are either commercially available or described in the literature.
Reduction of amide groups according to method d) is most conveniently performed with lithium aluminium hydride or alane in an inert organic solvent such as e.g. tetrahydrofuran or diethylether from 0 °C to reflux temperature.

The indole formation according to method e) is performed by the reaction of acetals of formula VII with aryl hydrazines of formula VIII resulting in the corresponding hydrazones, which subsequently are converted into indoles by means of the Fischer indole synthesis. The synthesis sequence is preferably performed as a one-pot procedure using a Lewis acid catalysts, preferably zinc chloride or boron fluoride, or protic acids, preferably sulfuric acid or phosphoric acid, in a suitable solvent such as acetic acid or ethanol at an elevated temperature.

Acetals of formula VII are prepared as indicated in the reaction scheme above using a mono substituted cyclic diamine of formula IX, wherein R⁰ is 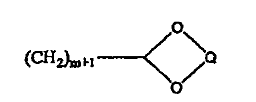 as key intermediates. The key intermediates of formula IX are prepared by alkylation of the cyclic diamine with an acetal of formula XIII 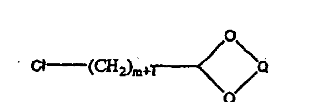 using the conditions described above for methods b.

Polymer bound acetals of formula XIII are prepared by reaction of aldehydes of formula Cl-(CH₂)ₘ₊₁CHO with commercially available 2,2-dimethyl-1,3-dioxolan-4-yl-methoxymethyl polystyrene in a suitable solvent such as toluene, using p-toluenesulfonic acid as catalyst at elevated temperature. 4-Chlorobutanal, 5-chloropentanal, and 6-chlorohexanal were prepared in analogy to the method described by Normant et al. *Tetrahedron* **1994,** *50* (40), 11665. Alternatively, the acetals of formula VII are prepared by alkylation of secondary amines of formula III with acetals of formula XIII using reaction conditions used for the alkylation according to method b, as described above.

### Experimental Section

All experiments were carried out under a positive pressure of nitrogen

Melting points were determined on a Büchi SMP-20 apparatus and are uncorrected. Mass spectra were obtained on a Quattro MS-MS system from VG Biotech, Fisons Instruments. Analytical LC-MS data were obtained on a PE Sciex API 150EX instrument equipped with IonSpray source and Shimadzu LC-8A/SLC-10A LC system. The LC conditions (50 X 4.6 mm YMC ODS-A with 5 µm particle size) were linear gradient elution with water/acetonitrile/trifluoroaectic acid (90:10:0.05) to water/acetonitrile/trifluoroacetic acid (10:90:0.03) in 7 min at 2 mL/min. Purity was determined by integration of the UV trace (254 nm). The retention times Rₜ are expressed in minutes. Preparative LC-MS-separation was performed on the same instrument. The LC conditions (50 X 20 mm YMC ODS-A with 5 µm particle size) were linear gradient elution with water/acetonitrile/trifluoroacetic acid (80:20:0.05) to water/acetonitrile/trifluoroacetic acid (10:90:0.03) in 7 min at 22.7 mL/min. Fraction collection was performed by split-flow MS detection. ¹H NMR spectra were recorded at 500.13 MHz on a Bruker Avance DRX500 instrument or at 250.13 MHz on a Bruker AC 250 instrument. Deuterated chloroform (99.8%D) or dimethyl sulfoxide (99.9%D) were used as solvents. TMS was used as internal reference standard. Chemical shift values are expressed in ppm-values. The following abbreviations arc used for multiplicity of NMR signals: s=singlet, d=doublet, t=triplet, q=quartet, qui=quintet, h=heptet, dd=double doublet, dt=double triplet, dq=double quartet, tt=triplet of triplets, m=multiplet and b=broad singulet. NMR signals corresponding to acidic protons are generally omitted. Content of water in crystalline compounds was determined by Karl Fischer titration. Standard workup procedures refer to extraction with the indicated organic solvent from proper aqueous solutions, drying of combined organic extracts (anhydrous MgSO₄ or Na₂SO₄), filtering and evaporation of the solvent *in vacuo*. For column chromatography silica gel of type Kieselgel 60, 230-400 mesh ASTM was used. For ion-exchange chromatography the following material was used: SCX-columns (1 g) from Varian Mega Bond Elut®, Chrompack cat. no. 220776. Prior use the SCX-columns were pre-conditioned with 10% solution of acetic acid in methanol (3 mL). For reversed phase chromatography the following material was used: C-18 columns (1 g) from Varian Mega Bond Elut®, Chrompack cat. no. 220508). Prior use the C-18-columns were pre-conditioned with methanol (3 mL) and water (3 mL). For decomplexation by irradiation an ultaviolet light source (300 W) from Philipps was used.

### Preparation of intermediates

### A. Preparation of arylpiperazines:

### η⁶-1,3-dichlorotoluene-η⁵-cyclopentadienyliron(II) hexafluorophosphate

Ferrocene (167 g), anhydrous aluminium trichloride (238 g) and powdered aluminium (24 g) were suspended in 1,3-dichlorotoluene (500 mL) and heated to 110 °C in a nitrogen atmosphere for 5 h. The mixture was cooled to room temperature and water (1000 mL) was added very carefully in small portions while cooling on an icebath. Heptane (500 mL) and diethyleher (500 mL) was added and the mixture stirred at room temperature for 30 min. The mixture was extracted with diethylether (3x 300 mL). Aqueous ammonium hexafluorophoshate (60 g in 50 mL water) was added in small portions to the remaining aqueous phase and the product allowed to precipitate at room temperature overnight. The precipitate was filtered off and dried to give 150 g (39%) product as a light green powder.

### 1-[2-Methyl-3-(morpholin-4-yl)phenyl]piperazine

η⁶-1,3-dichlorotoluene-η⁵-cyclopentadienyliron(II) hexafluorophosphate (21.3 g) was dissolved in dry THF (400 mL). Morpholine (8.7 mL) and anhydrous potassium carbonate (13.8 g) were added and the mixture stirred under nitrogen at room temperaturefor 72 h. Anhydrous piperazine (21.5 g) was added and the mixture heated to reflux for 24 h. The mixture was cooled to rt, filtered through celite and evaporated *in vacuo*. The brown solid was redissolved in a mixture of acetonitrile (250 mL) and concentrated ammonia (50 mL) followed by irradiation with a 500 w halogen lamp for 3 days. The mixture was filtered through celite and the volatile solvents evaporated *in vacuo*. The oily residue was resuspended in water (50 mL), the product filtered off, dried and recrystallised from acetone/heptane.

The following piperazine was prepared analogously:
1-[3-(2,6-Dimethylmorpholin-4-yl)-2-methylphenyl]piperazine
η⁶-[1-(3-Chloro-2-methylphenyl)-4-ethoxycarbonylpiperazine]-η⁵-cyclopentadienyliron(II) hexafluorophosphate
   η^{*6*}-1,3-dichlorotoluene-η^{*5*}-cyclopentadienyliron(II) hexafluorophosphate (25.6 g) and anhydrous potassium carbonate (16.6 g) were suspended in dry THF (600 mL). 1-Ethoxycarbonylpiperazine (19 g) was added dropwise under nitrogen at room temperature and the mixture was stirred under nitrogen at 30 °C for 72h. The mixture was filtered through celite and concentrated *in vacuo* to approximately 100 mL which was added to anhydrous diethylether (1000 mL) with vigorous stirring. The light green precipitate was collected by filtration and dried *in vacuo* to give 50 g of the product (95%).
1-(3-[3-(dimethylamino)phenoxy]-2-methylphenyl)piperazine
   Sodium hydride (0.6 g) was suspended in dry THF (20 mL). 3-(dimethylamino)phenol (1.8 g) was added in small portions (CAUTION: hydrogen gas is evolved) and the mixture stirred at room temperaturefor 15 min. η⁶-[1-(3-Chloro-2-methylphenyl)-4-ethoxycarbonylpiperazine]-η⁵-cyclopentadienyliron(II) hexafluorophosphate (5.5 g) was added and the mixture stirred at room temperature for 24 h. The volatile solvents were evaporated *in vacuo* and the solid residue was redissolved in acetonitrile (200 mL) and concentrated ammonia (50 mL). The resulting mixture was irradiated with a 500 w halogen lamp for 3 days. The mixture was filtered through celite and the volatile solvents evaporated *in vacuo*. The brown gum was boiled at reflux in concentrated hydrochloric acid for 24 h, cooled to room temperatureand added to 400 g ice. The mixture was made alkaline by the addition of concentrated sodium hydroxide (80 mL) and the precipated product was collected and dried *in vacuo* to give 3.5 g (95%) of the title compound.

The following piperazines were prepared analogously:
1-(2-Methyl-3-[3-(diethylamino)phenoxy]phenyl)piperazine.
1-[2-Methyl-3-(3,4,5-trimethoxyphenoxy)phenyl]piperazine.

### B. Preparation of haloalkylindoles.

### 3-(3-Bromopropyl)-1H-indole

Prepared as described in Crooks et al. *J. Med. Chem*. **1983**, *26*, 1470

### 3-(4-Bromobutyl)-1H-indole

Prepared as described in Crooks et al. *J. Med. Chem*. **1983**, *26*, 1470

### 3-(3-chloropropyl)-5-fluoro-1H-indole

5-Chloro-1-pentanol (16.2 mL) was dissolved in cold 5 mM solution of 2,2,6,6-tetramethylpiperidine-1-yloxy (tempo) in dichloromethane (240 mL) cooled to 0 °C. Potassium bromide (24 mL, 0.5 M in water) was added to the mixture. Sodium hydrogencarbonate (24 g) was dissolved in aqueous sodium hypochlorite (500 mL; 0.3 M) and the mixture added in one portion at 5°C under vigorous stiring. The mixture was stirred at 5°C for 20 min, the dichloromethane phase separated, and the water phase extracted with dichloromethane (200 mL). The organic phases were combined and the volatile solvents evaporated *in vacuo* giving 5-chloropentanal as a clear oil (16 g). 5-Chloropentanal was suspended in water (100 mL), 4-fluorophenylhydrazine hydrochloride (19.5 g) was added together with toluene (800 mL) and the mixture stirred at room temperature for 15 min.85% Phosphoric acid (100 mL) was added and the mixture boiled under reflux for 2 h. The mixture was cooled to rt, the toluene phase collected and washed with saturated aqueous sodium hydrogencarbonate, dried (MgSO₄) and the volatile solvents evaporated *in vacuo*. The oily residue was purified by flash chromatography on silicagel (eluent ethyl acetate:heptane; 1:4) to give the title compound as an orange oil (14 g, 56%).

The following indole was prepared analogously:
3-(4-chlorobutyl)-5-fluoro-1*H*-indole

### C. Preparation of solid-supported intermediates

### 2-(4-Chlorobutyl)-1,3-dioxolan-4-ylmethoxymethyl polystyrene.

A 2 L round bottom flask was charged with 2,2-dimethyl-1,3-dioxolan-4-ylmethoxymethyl polystyrene (90 g, 72 mmol, commercially available as (±)-1-(2,3-isopropylidene) glycerol polystyrene from Calbiochem-Novabiochem, cat. no. 01-64-0291). Toluene (900 mL) followed by p-toluenesulfonic acid mono hydrate (5.0 g, 26 mmol), sodium sulfate (25 g) and readily available 5-chloropentanal (25.5 g, 211 mmol) were added and the mixture boiled under reflux for 12 h. The reflux condenser was replaced by a Dean-Stark apparatus and the mixture was boiled under reflux for an additional 3 h. After cooling of the reaction mixture to 60 °C, the resin was filtered off and washed with toluene (200 mL), tetrahydrofuran/pyridine (1:1, 200 mL), tetrahydrofuran/ water/pyridine (10:10:1, 200 mL), methanol (200 mL), water (200 mL), tetrahydrofuran (200 mL), dichloromethane (200 mL), methanol (3 X 200 mL) and dichloromethane (3 X 200 mL). The resin was dried *in vacuo* (55 °C, 12 h) to yield the title compound (97 g).

The following compounds were prepared analogously:
2-(3-Chloropropyl)-1,3-dioxolan-4-ylmethoxymethyl polystyrene
2-(5-Chloropentyl)-1,3-dioxolan-4-ylmethoxymethyl polystyrene

### Preparation of the compounds of the invention

### Example 1

### 1-(2-methyl-3-[3-(dimethylamino)phenoxy]phenyl)-4-[3-(1H-indol-3-yl)-propyl]piperazine (1a),

3-(3-Bromopropyl)-1*H*-indole (2 g), potassium carbonate (1.8 g) and 1-(3-[3-(dimethylamino)phenyloxy]-2-methylphenyl)piperazine (2 g) were mixed in anhydrous acetonitrile and boiled under reflux for 5 h. After cooling to room temperature, silicagel (7g) was added and the volatile solvents evaporated *in vacuo*. The compound was purified by flash chromatography on silicagel using ethyl acetate:heptane:triethylamine (49:49:2) as eluent. Fractions containing the compound were combined and concentrated *in vacuo*. Recrystallisation from acetonitrile gave the title compound as white crystals. 2 g (66%). Mp 104-105°C. ¹H NMR (DMSO-d₆): 1.80-1.70 (m, 2H); 2.15 (s, 3H); 2.40 (t, 2H); 2.70 (t, 2H); 2.90-2.80 (m, 8H); 3.35 (s, 6H); 6.03 (d, 1H); 6.30 (s, 1H); 6.43 (m, 1H); 6.55 (d, 1H); 6.85 (d, 1H); 7.10-6.95 (m, 5H); 7.35 (d, 1H); 7.50 (d, 1H); 10.75 (br. S, 1H). Analysis (C₃₀H₃₆N₄O), calcd. C:76.89, H: 7.74, N: 11.95; found: C: 76.95, H:7.75, N: 12.01.Ms m/z: 469.3 (MH⁺).

The following compounds were prepared analogously:
*1-[3-(1H-Indol-3-yl)propyl]-4-[2-methyl-3-(morpholin-4-yl)-phenyl]piperazine* (**1b**), Mp 112-113°C. ¹H NMR (DMSO-d₆): 1.80-1.70 (m, 2H); 2.20 (s, 3H), 2.40 (t, 2H); 2.50 (m, 4H); 2.70 (t, 2H); 2.90-2.80 (m, 8H); 3.70 (m, 4H); 6.70 (m, 2H); 7.10-6.95 (m, 4H); 7.35 (d, 1H); 7.50 (d, 1H); 10.75 (br. S, 1H). Analysis (C₂₆H₃₄N₄O, ½H₂O) calcd. C:73.03, H: 8.25, N: 13.10; found: C: 72.67, H:7.94, N: 13.03.Ms m/z: 419.3 (MH⁺).
*1-[2-methyl-3-(morpholin-4-yl)phenyl]-4-[4-(1H-indol-3-yl)butyl]piperazine* (**1c**), Mp 111-113°C. ¹H NMR (DMSO-d₆): 1.60-1.50 (m, 2H); 1.80-1.70 (m, 2H); 2.20 (s, 3H); 2.40 (t, 2H); 2.50 (m, 4H); 2.70 (t, 2H); 2.90-2.80 (m, 8H); 3.70 (m, 4H); 6.70 (m, 2H); 7.10-6.95 (m, 4H); 7.30 (d, 1H); 7.50 (d, 1H); 10.75 (br. S, 1H). Analysis (C₂₇H₃₆N₄O) calcd. C: 74.96, H: 8.39, N: 12.95; found: C: 74.52, H: 8.34, N: 13.02.Ms m/z: 433.6 (MH⁺).
*1-[2-methyl-3-(morpholin-4-yl)phenyl]-4-[3-(5-fluoro-1H-indol-3-yl)propyl]piperazine* (**1d**),
   (44%). Mp 212-215°C. ¹H NMR (DMSO-d₆): 2.20 (m, 2H); 2.30 (s, 3H); 2.70 (t, 2H); 3.0-2.90 (m, 4H); 3.30 (m, 8H); 3.50 (m, 2H); 3.85 (m, 4H); 6.90-6.65 (m, 3H); 7.17 (m, 1H); 7.35-7.25 (m, 3H); 10.75 (br. S, 1H). Analysis (C₂₆H₃₃FN₄O, 3 HCl) calcd. C: 57.20, H: 6.65, N: 10.26; found: C: 56.81, H: 6.92, N: 9.96.Ms m/z: 437.4 (MH⁺).
*1-[2-methyl-3-(morpholin-4-yl)phenyl]-4-[4-(5-fluoro-1H-indol-3-yl)butyl]piperazine* (**1e**)
   Mp 112-114°C. ¹H NMR (DMSO-d₆): 1.60-1.50 (m, 2H); 1.80-1.70 (m, 2H); 2.20 (s, 3H); 2.35 (t, 2H); 2.50 (m, 4H); 2.70 (t, 2H); 2.80 (m, 8H); 3.75 (m, 4H); 6.70 (m, 2H); 6.85 (m, 1H); 7.10 (t, 1H); 7.20 (s, 1H); 7.25 (m, 1H); 7.30 (m, 1H); 10.75 (br. S, 1H). Analysis (C₂₇H₃₅FN₄O) calcd. C: 71.97, H: 7.83, N: 12.43; found: C: 70.71, H: 7.91, N: 12.28. Ms m/z: 451.5 (MH⁺).
*1-[3-(5-chloro-1H-Indol-3-yl)propyl]-4-[2-methyl-3-(morpholin-4-yl)-phenyl]piperazine* (**1d**)
   ¹H NMR (DMSO-d₆): 1.85-1.75 (m, 2H); 2.23 (s, 3H); 2.40 (t, 2H); 2.55 (m, 4H); 2.70 (t, 2H); 2.80 (m, 4H); 2.85 (m, 4H); 3.75 (m, 4H); 6.75 (m, 2H); 7.05 (m, 1H); 7.10 (m, 1H); 7.25 (s, 1H); 7.30 (d, 1H); 7.55 (s, 1H); 10.95 (br. S, 1H). Ms m/z: 453.5 (MH⁺).

### Example 2

### 1-{3-[3-(Diethylamino)phenoxy]-2-methylphenyl}-4-[3-(5-fluoro-1H-indol-3-yl)propyl]piperazine (2a).

2-(3-Chlorobutyl)-1,3-dioxolan-4-ylmethoxymethyl polystyrene (70 g, 90.3 mmol) was suspended in dry N,N-dimethylformamide (700 mL). Sodium iodide (68 g, 452 mmol) was added followed by diisopropylethylamine (232 mL, 1.36 mol) and piperazine (117 g, 1.36 mol). The reaction mixture was heated at 80 °C under stirring for 12 h. After cooling to room temperature, the resin was filtered off and washed with N,N-dimethylformamide (3 X 500 mL), methanol (3 X 500 mL), tetrahydrofuran (3 X 500 mL) and subsequently with methanol and tetrahydrofuran (each 250 mL, 5 cycles). Finally, the resin was washed with dichloromethane (3 X 500 mL) and dried *in vacuo* (25 °C, 36 h) to yield an almost colourless resin (76 g).

A part of the resin obtained (12.8 g, 16.6 mmol) was suspended in (5:1)-mixture of dry tetrahydrofuran/N,N-dimethylformamide (150 mL). η⁶-2,6-Dichlorotoluene-η⁵-cyclopentadienyliron(II) hexafluorophosphate (12.5 g, 29.3 mmol) was added followed by potassium carbonate (8.8 g, 63.7 mmol). The reaction mixture was stirred at 60 °C for 12 h. After cooling to room temperature, the resin was filtered off and washed with tetrahydrofuran (2 X 100 mL), water (2 X 50 mL), tetrahydrofuran (2 X 100 mL), methanol (2 X 50 mL), dichloromethane (2 X 100 mL), methanol (2 X 50 mL). Finally, the resin was washed with dichloromethane (3 X 100 mL) and dried *in vacuo* (25 °C, 36 h) to yield a dark orange resin (17.5 g).

To a solution of 3-(diethylamino)-phenol (1.1 g, 6.8 mmol) in tetrahydrofuran (25 mL) was carefully added neat sodium hydride (6.2 mmol) at room temperature (Caution: Generation of hydrogen). The mixture was stirred for additional 30 min after the generation of hydrogen ceased. Subsequently, a part of the above obtained resin (1.1 g, 0.94 mmol) was added and the mixture was stirred at 40 °C for 12 h. After cooling to room temperature, the resin was filtered off and washed with tetrahydrofuran (2 X 25 mL), tetrahydrofuran/water (1:1) (2 X 25 mL), N,N-dimethylformamide (2 X 25 mL), water (2 X 25 mL), methanol (3 X 25 mL), tetrahydrofuran (3 X 25 mL) and subsequently with methanol and tetrahydrofuran (each 25 mL, 5 cycles). Finally, the resin was washed with dichloromethane (3 X 25 mL) and dried *in vacuo* (25 °C, 12 h).

A part of the obtained resin (200 mg, 0.15 mmol) and a 0.5 M solution of 1,10-phenanthroline in a (3:1)-mixture of pyridine/water (10 mL) was placed in a light-transparent reactor tube. The suspension was vortexed and irradiated with visible light for 12 h. A very characteristic feature of the decomplexation step is the appearance of the intensive red colour of the liquid phase during irradiation. The resin was filtered and washed with methanol (2 X 10 mL), water (2 X 10 ml) and tetrahydrofuran (3 X 10 mL) until the washing solution kept colourless (approximately 5 cycles) and the irradiation procedure was repeated until decomplexation was complete (approximately 4 cycles). After complete decomplexation, the resin was washed with dichloromethane (3 X 10 mL) and dried *in vacuo* (25 °C, 12 h).

The obtained resin (154 mg, 0.15 mmol) and 4-fluorophenylhydrazine hydrochloride (35 mg, 0.21 mmol) were mixed in a reactor tube. A 0.5 M solution of anhydrous zinc chloride in acetic acid (1.5 mL) was added and the reaction tube was sealed. The reaction mixture was stirred for 12 h at 70 °C. After cooling to room temperature, the reaction mixture was filtered and the residual resin washed with dimethylsulfoxide (1.5 mL). To the combined filtrates, a saturated aqueous sodium carbonate solution (1.5 mL) was carefully added (Caution: Generation of carbondioxide). The solution was loaded on a on a pre-conditioned reversed phase C-18 column. The column was washed with water (4 mL) and the product was eluted with methanol (4.5 mL). After evaporation of the volatile solvents, the crude product was purified by preparative reversed phase HPLC chromatography. The resulting solution was subsequently loaded on a pre-conditioned ion exchange column. The column was washed with methanol (4 mL) and acetonitrile (4 mL), followed by elution of the product with 4 N solution of ammonia in methanol (4.5 mL). Evaporation of the volatile solvents afforded the title compound as yellow oil (5 mg, 9.7 µmol ). LC/MS (m/z) 515 (MH⁺). Rt = 3.46, purity: 82%.

The following compounds were prepared analogously:
*1-[2-Methyl-3-(3,4,5-trimethoxyphenoxy)phenyl]-4-[3-(5-fluoro-1H-indol-3-yl)propyl]piperazine* (**2b**): LC/MS (m/z) 534 (MH⁺), RT = 4.34, purity: 84%.
*1-{3-[3-(Diethylamino)phenoxy]-2-methylphenyl}-4-[3-(5-chloro-1H-indol-3-yl)propyl]piperazine* (**2c**): LC/MS (m/z) 531 (MH⁺), RT = 3.62, purity: 80%.
*1-{3-[3-(Diethylamino)phenoxy]-2-methylphenyl}-4-[3-(5,7-difluoro-1H-indol-3-yl)propyl]piperazine* (**2d**): LC/MS (m/z) 533 (MH⁺), RT = 3.56, purity: 71%.
*1-[2-Methyl-3-(3,4,5-trimethoxyphenoxy)phenyl}-4-[3-(5-chloro-1H-indol-3-yl)propyl]piperazine* (**2e**): LC/MS (m/z) 550 (MH⁺), RT = 4.51, purity: 77%.
*1-{3-[3-(Diethylamino)phenoxy]-2-methylphenyl}-4-[3-(1H-indol-3-yl)propyl]piperazine* (**2f**): LC/MS (m/z) 497 (MH⁺), RT = 3.28, purity: 74%.
*1-[3-(2,6-Dimethylmorpholin-4-yl)-2-methylphenyl]-4-[3-(5-fluoro-1H-indol-3-yl)propyl]-1,4-diazepan* (**2g**): LC/MS (m/z) 479 (MH⁺), RT = 4.29, purity: 91%.
*1-{3-[3-(Dimethylamino)phenoxy]-2-methylphenyl}-4-[3-(5-fluoro-1H-indol-3-yl)propyl]piperazine* (**2h**): LC/MS (m/z) 487 (MH⁺), RT = 3.71, purity: 83%.
*1-[3-(1,3-Benzodioxolan-5-yloxy)-2-methylphenyl]-4-[3-(1H-indol-3-yl)propyl]piperazine* (**2i**): LC/MS (m/z) 470 (MH⁺), RT = 4.40, purity: 77%.
*1-[3-(2,6-Dimethylmorpholin-4-yl)-2-methylphenyl]-4-[3-(5-fluoro-1H-indol-3-yl)propyl]piperazine* (**2j**): LC/MS (m/z) 465 (MH⁺), RT = 4.14, purity: 81%:
*1-{3-[3-(Diethylamino)phenoxy]-2-methylphenyl}-4-[4-(5-fluoro-1H-indol-3-yl)butyl]piperazine* (**2k**): LC/MS (m/z) 5295 (MH⁺), RT = 3.50, purity: 70%.
*1-[3-(Morpholin-4-yl)-2-methylphenyl]-4-[3-(5-chloro-1H-indol-3-yl)propyl]piperazine* (**2l**): LC/MS (m/z) 453 (MH⁺), RT = 3.97, purity: 84%.
*1-{3-[3-(Dimethylamino)phenoxy]-2-methylphenyl}-4-[3-(5-chloro-1H-indol-3-yl)propyl]piperazine* (**2m**): LC/MS (m/z) 503 (MH⁺), RT = 3.96, purity: 87%.
*(S)-1-[3-(2-Hydroxymethylpyrrolidin-1-yl)-2-methylphenyl]-4-[3-(1H-indol-3-yl)propyl]piperazine* (**2o**): LC/MS (m/z) 433 (MH⁺), RT = 2.33, purity: 85%.
*(S)-1-[3-(2-Hydroxymethylpyrrolidin-1-yl)-2-methylphenyl]-4-[3-(5-fluoro-1H-indol-3-yl)propyl]piperazine* (**2p**): LC/MS (m/z) 451.3 (MH⁺), RT = 2.50, purity: 81 %.
*1-[3-(1,3-Benzodioxolan-5-yloxy)-2-methylphenyl]-4-[3-(5-fluoro-1H-indol-3-yl)propyl]piperazine* (**2q**): LC/MS (m/z) 488 (MH⁺), RT = 4.48, purity: 98%.
*1-{3-[3-(Dimethylamino)phenoxy]-2-methylphenyl}-4-[4-(5,7-difluoro-1H-indol-3-yl)butyl]piperazine* (**2r**): LC/MS (m/z) 519 (MH⁺), RT = 4.09, purity: 70%.
*(S)-1-[3-(2-Hydroxymethylpyrrolidin-1-yl)-2-methylphenyl]-4-[3-(5,7-difluoro-1H-indol-3-yl)propyl]piperazine* (**2s**): LC/MS (m/z) 469 (MH⁺), RT = 2.67, purity: 75%.
*1-[3-(1,3-Benzodioxolan-5-yloxy)-2-methylphenyl]-4-[3-(5,7-difluoro-1H-indol-3-yl)propyl]piperazine* (**2t**): LC/MS (m/z) 506 (MH⁺), RT = 4.60, purity: 70%.
*1-[3-(2,6-Dimethylmorpholin-4-yl)-2-methylphenyl]-4-[3-(1H-indol-3-yl)propyl]-1,4-diazepane* (**2v**): LC/MS (m/z) 461 (MH⁺), RT = 4.20, purity: 83%.
*1-[3-(2,6-Dimethylmorpholin-4-yl)-2-methylphenyl]-4-[3-(5,7-difluoro-1H-indol-3-yl)propyl]piperazine* (**2w**): LC/MS (m/z) 483 (MH⁺), RT = 4.29, purity: 75%.
*1-{3-[3-(Diethylamino)phenoxy]-2-methylphenyl}-4-[3-(5,7-difluoro-1H-indol-3-yl)propyl]-1,4-diazepane* (**2x**): LC/MS (m/z) 547 (MH⁺), RT = 3.61, purity: 76%.
*1-{3-[3-(Dimethylamino)phenoxy]-2-methylphenyl}-4-[3-(1H-indol-3-yl)propyl]]-1,4-diazepane* (**2y**): LC/MS (m/z) 483 (MH⁺), RT = 3.78, purity: 72%.
*1-{3-[3-(Dimethylamino)phenoxy]-2-methylphenyl}-4-[3-(5,7-difluoro-1H-indol-3-yl)propyl]piperazine* (**2z**): LC/MS (m/z) 505 (MH⁺), RT = 3.94, purity: 85%.
*1-[3-(Morpholin-4-yl)-2-methylphenyl]-4-[3-(5-fluoro-1H-indol-3-yl)propyl]-1,4- diazepane* (**2aa**): LC/MS (m/z) 451 (MH⁺), RT = 3.82, purity: 88%.
*1-{3-[3-(Diethylamino)phenoxy]-2-methylphenyl}-4-[4-(5-chloro-1H-indol-3-yl)butyl]piperazine* (**2ab**): LC/MS (m/z) 545 (MH⁺), RT = 3.77, purity: 82%.
*1-[3-(1,3-Benzodioxolan-5-yloxy)-2-methylphenyl]-4-[3-(5-fluoro-1H-indol-3-yl)propyl]-1,4-diazepane* (**2ac**): LC/MS (m/z) 502 (MH⁺), RT = 4.56, purity: 75%.
*1-{3-[3-(Diethylamino)phenoxy]-2-methylphenyl}-4-[4-(5,7-difluoro-1H-indol-3-yl)butyl]piperazine* (**2ad**); LC/MS (m/z) 547 (MH⁺), RT = 3.69, purity: 87%.
*1-{3-[3-(Morpholin-4-yl)phenoxy]-2-methylphenyl}-4-[3-(5-fluoro-1H-indol-3-yl)propyl]-1,4-diazepane* (**2ae**): LC/MS (m/z) 543 (MH⁺), RT = 4.45, purity: 75%.
*(S)-1-[3-(2-Hydroxymethylpyrrolidin-1-yl)-2-methylphenyl]-4-[3-(5-chloro-1H-indol-3-yl)propyl]piperazine* (**2af**): LC/MS (m/z) 467 (MH⁺), RT = 2.75, purity: 70%.
*1-[3-(1,3-Benzodioxolan-5-yloxy)-2-methylphenyl]-4-[3-(5-chloro-1H-indol-3-yl)propyl]-1,4-diazepane* (**2ag**) LC/MS (m/z) 504 (MH⁺), RT = 4.66, purity: 74%.
*1-[3-(2,6-Dimethylmorpholin-4-yl)-2-methylphenyl]-4-[3-(5,7-difluoro-1H-indol-3-yl)propyl]-1,4-diazepane* (**2ag**): LC/MS (m/z) 497 (MH⁺), RT = 4.42, purity: 76%.

### Pharmacological Testing

The compounds of the invention were tested according to well recognised and reliable methods. The tests were as follows:

### Inhibition of the binding of ³H-YM-09151-2 to human dopamine D₄ receptors

By this method the inhibition by drugs of the binding of [³H]YM-09151-2 (0.06 nM) to membranes of human cloned dopamine D_{4.2} receptors expressed in CHO-cells is determined *in vitro*. Method modified from NEN Life Science Products, Inc., technical data certificate PC2533-10/96. The results are given in the following Table 1 as IC₅₀-values or as percent inhibition of the binding of ³H-YM-09151-2 at the concentration of the test compound indicated.

### Inhibition of the binding of [³H]-Spiperone to human D₃ receptors

By this method the inhibition by drugs of the binding [³H]Spiperone (0.3 nM) to membranes of human cloned dopamine D₃ receptors expressed in CHO-cells is determined *in vitro*. Method modified from R.G. MacKenzie et al. *Eur. J. Pharm.-Mol. Pharm. Sec.* **1994**, *266*, 79-85. The results are given in the following Table 1 as IC₅₀-values or as percent inhibition of the binding of ³H-Spiperone at the concentration of the test compound indicated.

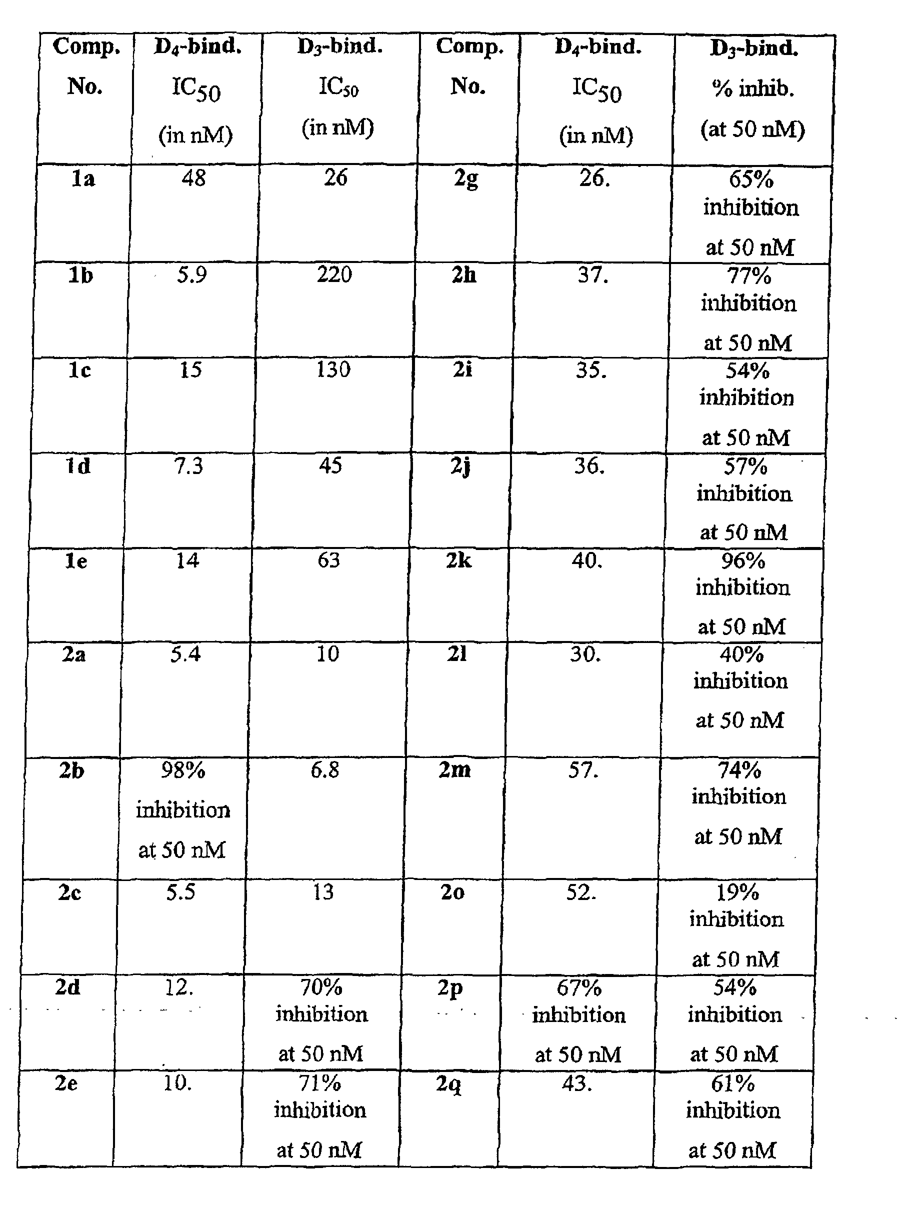

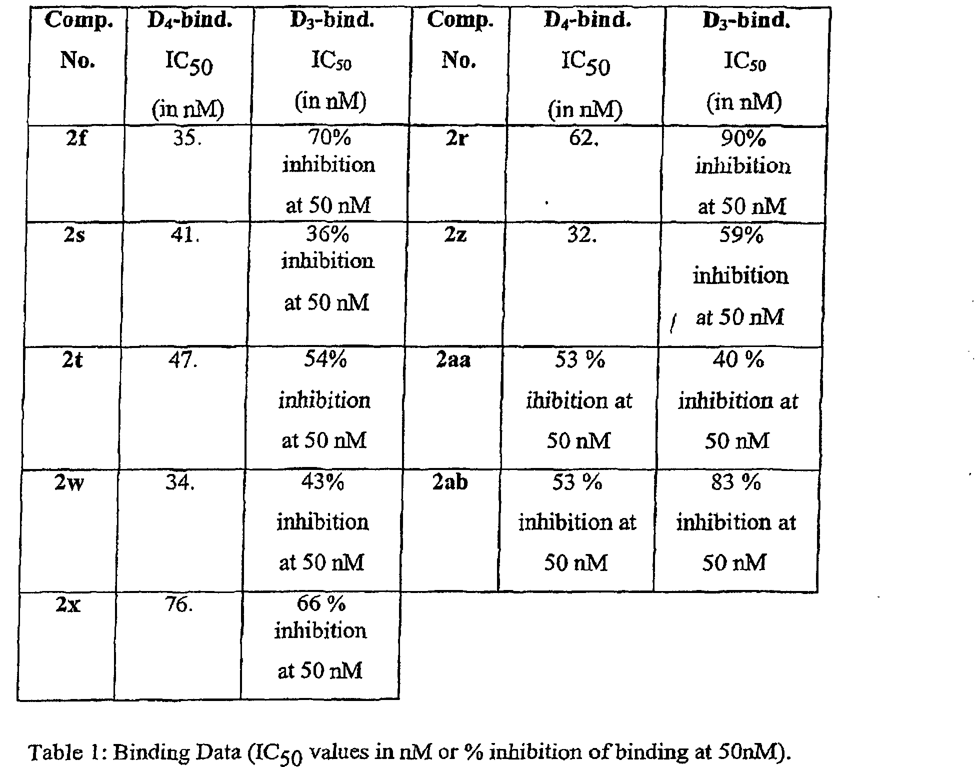

In general, the compounds of the invention have been found potently to inhibit the binding of tritiated YM-09151-2 to dopamine D₄ receptors. Many of the compounds also inhibit binding of [³H]-Spiperone to dopamine D₃ receptors.
The compounds have also been tested in a functional assay described in *Br. J. Pharmacol*. **1999**, *128*, 613-629. In this test, the compounds were shown to be partial agonists or antagonists at the dopamine D₄ receptors.

The compounds have only weak or no affinity for the dopamine D₂ receptor.
In addition some of the compounds have 5-HT reuptake inhibiting effect and some of the compounds inhibit the binding to scrotonergic receptors.

Accordingly, the compounds of the invention are considered useful in the treatment of psychosis, the positive and negative symptoms of schizophrenia and affective disorders, such as generalised anxiety disorder, panic disorder, obsessive compulsive disorder, depression and aggression.

The pharmaceutical compositions of this invention or those which are manufactured in accordance with this invention may be administered by any suitable route, for example orally in the form of tablets, capsules, powders, syrups, etc., or parenterally in the form of solutions for injection. For preparing such compositions, methods well known in the art may be used, and any pharmaceutically acceptable carriers, diluents, excipients or other additives normally used in the art may be used.

Conveniently, the compounds of the invention are administered in unit dosage form containing said compounds in an amount of about 0.01 to 100 mg.

The total daily dose is usually in the range of about 0.05 - 500 mg, and most preferably about 0.1 to 50 mg of the active compound of the invention.

### Formulation Examples

The pharmaceutical formulations of the invention may be prepared by conventional methods in the art.

For example: Tablets may be prepared by mixing the active ingredient with ordinary adjuvants and/or diluents and subsequently compressing the mixture in a conventional tabletting machine. Examples of adjuvants or dilucnts comprise: corn starch, potato starch, talcum, magnesium stearate, gelatine, lactose, gums, and the like. Any other adjuvants or additives such as colourings, flavourings, preservatives etc. may be used provided that they are compatible with the active ingredients.
Solutions for injections may be prepared by dissolving the active ingredient and possible additives in a part of the solvent for injection, preferably sterile water, adjusting the solution to desired volume, sterilisation of the solution and filling in suitable ampules or vials. Any suitable additive conventionally used in the art may be added, such as tonicity agents, preservatives, antioxidants, etc.
Typical examples of recipes for the formulation of the invention are as follows:
1) Tablets containing 5.0 mg of a compound of the invention calculated as the free base:

| | |
|---|---|
| Active compound | 5.0 mg |
| Lactose | 60 mg |
| Maize starch | 30 mg |
| Hydroxypropylcellulose | 2.4 mg |
| Microcrystalline cellulose | 19.2 mg |
| Croscarmellose Sodium Type A | 2.4 mg |
| Magnesium stearate | 0.84 mg |

2) Tablets containing 0.5 mg of a compound of the invention calculated as the free base:

| | |
|---|---|
| Active compound | 0.5 mg |
| Lactose | 46.9 mg |
| Maize starch | 23.5 mg |
| Povidone | 1.8 mg |
| Microcrystalline cellulose | 14.4 mg |
| Croscarmellose Sodium Type A | 1.8 mg |
| Magnesium stearate | 0.63 mg |

3) Syrup containing per millilitre:

| | |
|---|---|
| Active compound | 25 mg |
| Sorbitol | 500 mg |
| Hydroxypropylcellulose | 15 mg |
| Glycerol | 50 mg |
| Methyl-paraben | 1 mg |
| Propyl-paraben | 0.1 mg |
| Ethanol | 0.005 ml |
| Flavour | 0.05 mg |
| Saccharin sodium | 0.5 mg |
| Water | ad 1 ml |

4) Solution for injection containing per millilitre:

| | |
|---|---|
| Active compound | 0.5 mg |
| Sorbitol | 5.1 mg |
| Acetic Acid | 0.05 mg |
| Saccharin sodium | 0.5 mg |
| Water | ad 1 ml |

## Claims

1. A phenylpiperazinyl do derivative having the formula 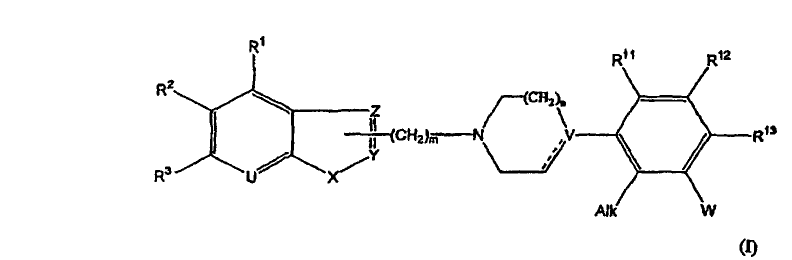 wherein X is N, NR, CHR, CR, O or S, Z and Y are selected from N and C, CR, and R is hydrogen, alkyl or acyl; provided that at least one of X, Y and Z is a heteroatom;
U is selected from CR⁴ and N;
V is C, CH or N, and the dotted line emanating from V indicates a bond when V is C and no bond when V is N or CH;
Alk is C₁₋₆-alkyl;
W is a ring of formula -NR¹⁴R¹⁵ wherein R¹⁴ and R¹⁵ together with the nitrogen atom to which they are attached form a 5 to 6 membered ring, which may contain an additional heteroatom selected from N, O and S, and which may be substituted one or more times with substituents selected from halogen, trifluoromethyl, nitro, cyano, C₁- ₆-alkyl, C₂₋₆-alkenyl, C₂₋₆-alkynyl, C₁₋₆-alkoxy, C₁₋₆-alkylthio, C₁₋₆-alkylsulfonyl, hydroxy, hydroxy-C₁₋₆ alkyl, amino, C₁₋₆-alkylamino, di-(C₁₋₆-alkyl)amino, acyl, aminocarbonyl, C₁₋₆-alkylaminocarbonyl, and a di-(C₁₋₆-alkyl)aminocarbonyl; or W is a phenoxy or phenylsulfanyl group, which may be substituted one or more times with substituents selected from halogen, trifluoromethyl, nitro, cyano, C₁₋₆-alkyl, C₂₋₆-alkenyl, C₂₋₆-alkynyl, C₁₋₆-alkoxy, C₁₋₆-alkylthio, C₁₋₆-alkylsulfonyl, hydroxy, hydroxy-C₁₋₆ alkyl, amino, C₁₋₆-alkylamino, di-(C₁₋₆-alkyl)amino, acyl, aminocarbonyl, C₁₋₆-alkylaminocarbonyl, di-(C₁₋₆-alkyl)aminocarbonyl and a ethylene dioxy- and a methylenedioxy group;
R¹, R², R³, R⁴, R¹¹, R¹² and R¹³ is selected from hydrogen, halogen, trifluoromethyl, nitro, cyano, C₁₋₆-alkyl, C₂₋₆-alkenyl, C₂₋₆-alkynyl, C₁₋₆-alkoxy, C₁₋₆-alkylthio, C₁₋₆-alkylsulfonyl, hydroxy, hydroxy-C₁₋₆ alkyl, amino, C₁₋₆-alkylamino, di-(C₁₋₆-alkyl)amino, acyl, and aminocarbonyl, C₁₋₆-alkylaminocarbonyl and di-(C₁₋₆-alkyl)aminocarbonyl;
n is 1, or 2; m is 1, 2, 3, 4, 5 or 6;
and acid addition salts thereof.

2. A compound according to claim 1 wherein W is an optionally substituted morpholino, piperidinyl or pyrrolyl group.

3. A compound according to claim 2 wherein W is optionally substituted morpholino group.

4. A compound according to claim I wherein W is an optionally substituted phenoxy or phenylsulfanyl group.

5. A compound according to claim 1 wherein U is CR⁴, X is NR, Y is CH, Z is C and the thus formed indole is attached to the remainder of the molecule via position 3.

6. A compound according to claims 1-5 wherein V is N.

7. A compound according to claims 1 to 6 wherein m is 3, 4, 5 or 6.

8. A compound according to claim 1 wherein Alk is methyl.

9. A pharmaceutical composition **characterised in that** it comprises a compound of any of claims 1 to 8 together with one or more pharmaceutically acceptable carriers or diluents.

10. Use of a compound of any of claims 1 to 8 for the manufacture of a medicament useful in the treatment of psychosis, the positive and negative symptoms of schizophrenia and affective disorders, such as generalised anxiety disorder, panic disorder, obsessive compulsive disorder, depression and aggression.

## Patentansprüche

1. Phenylpiperazinyl-, -piperidinyl- und -tetrahydropyridinyl-Derivat mit der Formel: 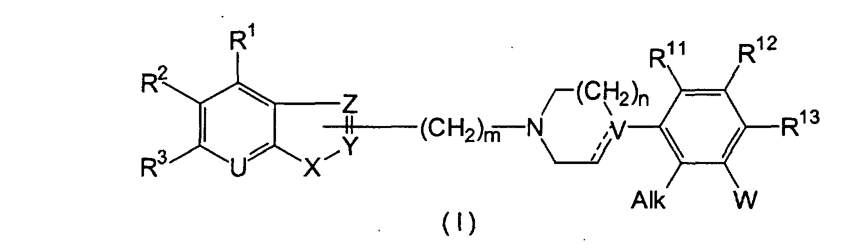 worin X N, NR, CHR, CR, O oder S ist, Z und Y aus N, C und CR ausgewählt sind und R Wasserstoff, Alkyl oder Acyl ist; mit der Massgabe, dass wenigstens eines aus X, Y und Z ein Heteroatom ist;
U aus CR⁴ und N ausgewählt ist;
V C, CH oder N ist und die gestrichelte Linie, die von V ausgeht, eine Bindung anzeigt, wenn V C ist, und keine Bindung, wenn V N oder CH ist;
Alk C₁₋₆-Alkyl ist;
W ein Ring der Formel -NR¹⁴R¹⁵ ist, worin R¹⁴ und R¹⁵ zusammen mit dem Stickstoffatom, an das sie gebunden sind, einen 5- bis 6-gliedrigen Ring bilden, der ein zusätzliches, aus N, O und S ausgewähltes Heteroatom enthalten kann, und der ein- oder mehrmals mit Substituenten substituiert sein kann, die aus Halogen, Trifluormethyl, Nitro, Cyano, C₁₋₆-Alkyl, C₂₋₆-Alkenyl, C₂₋₆-Alkinyl, C₁₋₆-Alkoxy, C₁₋₆-Alkylthio, C₁₋₆-Alkylsulfonyl, Hydroxy, Hydroxy-C₁₋₆-alkyl, Amino, C₁₋₆-Alkylamino, Di-(C₁₋₆-alkyl)amino, Acyl, Aminocarbonyl, C₁₋₆-Alkylaminocarbonyl und Di(C₁₋₆-alkyl)aminocarbonyl ausgewählt sind; oder W eine Phenoxy- oder Phenylsulfanylgruppe ist, die ein- oder mehrmals mit Substituenten substituiert sein kann, die aus Halogen, Trifluormethyl, Nitro, Cyano, C₁₋₆-Alkyl, C₂₋₆-Alkenyl, C₂₋₆-Alkinyl, C₁₋₆-Alkoxy, C₁₋₆-Alkylthio, C₁₋₆-Alkylsulfonyl, Hydroxy, Hydroxy-C₁₋₆-alkyl, Amino, C₁₋₆-Alkylamino, Di-(C₁₋₆-alkyl)amino, Acyl, Aminocarbonyl, C₁₋₆-Alkylaminocarbonyl, Di-(C₁₋₆-alkyl)aminocarbonyl und einer Ethylendioxy- und einer Methylendioxygruppe ausgewählt sind;
R¹, R², R³, R⁴, R¹¹, R¹² und R¹³ aus Wasserstoff, Halogen, Trifluormethyl, Nitro, Cyano, C₁₋₆-Alkyl, C₂₋₆-Alkenyl, C₂₋₆-Alkinyl, C₁₋₆-Alkoxy, C₁₋₆-Alkylthio, C₁₋₆-Alkylsulfonyl, Hydroxy, Hydroxy-C₁₋₆-alkyl, Amino, C₁₋₆-Alkylamino, Di-(C₁₋₆-alkyl)amino, Acyl, Aminocarbonyl, C₁₋₆-Alkylaminocarbonyl und Di-(C₁₋₆-alkyl)aminocarbonyl ausgewählt sind;
n 1 oder 2 ist; m 1, 2, 3, 4, 5 oder 6 ist;
und Säureadditionssalze davon.

2. Verbindung gemäss Anspruch 1, worin W eine gegebenenfalls substituierte Morpholino-, Piperidinyl- oder Pyrrolylgruppe ist.

3. Verbindung gemäss Anspruch 2, worin W eine gegebenenfalls substituierte Morpholinogruppe ist.

4. Verbindung gemäss Anspruch 1, worin W eine gegebenenfalls substituierte Phenoxy- oder Phenylsulfanylgruppe ist.

5. Verbindung gemäss Anspruch 1, worin U CR⁴ ist, X NR ist, Y CH ist, Z C ist und das so gebildete Indol an den Rest des Moleküls über die Position 3 gebunden ist.

6. Verbindung gemäss Ansprüchen 1 bis 5, worin V N ist.

7. Verbindung gemäss Ansprüchen 1 bis 6, worin m 3, 4, 5 oder 6 ist.

8. Verbindung gemäss Anspruch 1, worin Alk Methyl ist.

9. Pharmazeutische Zusammensetzung, **dadurch gekennzeichnet, dass** sie eine Verbindung gemäss einem der Ansprüche 1 bis 8 zusammen mit einem oder mehreren pharmazeutisch akzeptablen Trägern oder Verdünnungsmitteln umfasst.

10. Verwendung einer Verbindung gemäss einem der Ansprüche 1 bis 8 zur Herstellung eines Medikaments, das in der Behandlung von Psychose, der positiven und negativen Symptome von Schizophrenie und affektiven Störungen, wie allgemeine Angststörung, Panikstörung, Zwangsstörung, Depression und Aggression, nützlich ist.

## Revendications

1. Dérivé de phénylpipérazinyle, -pipéridinyle et -tétrahydropyridinyle de formule 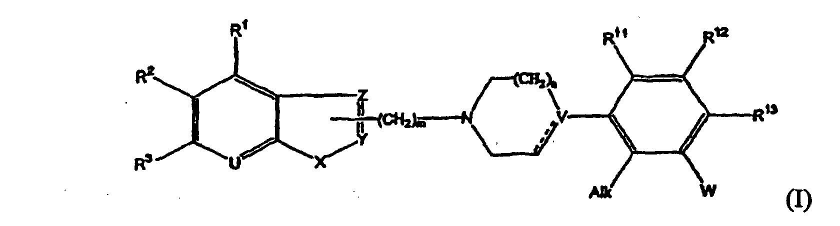 dans laquelle X est N, NR, CHR, CR, O ou S, Z et Y sont choisis parmi N, C et CR, et R est un atome d'hydrogène ou un groupe alkyle ou acyle, à condition qu'au moins l'un des X, Y et Z soit un hétéroatome;
U est choisi parmi CR⁴ et N;
V est C, CH ou N, et la ligne pointillée partant de V indique une liaison lorsque V est C et l'absence de liaison lorsque V est N ou CH;
Alk est un groupe alkyle en C₁-C₆;
W est un cycle de formule -NR¹⁴R¹⁵ où R¹⁴ et R¹⁵ forment ensemble, avec l'atome d'azote auquel ils sont liés, un cycle de 5 à 6 chaînons pouvant contenir un hétéroatome supplémentaire choisi parmi N, O et S, et pouvant être substitué une ou plusieurs fois par des substituants choisis parmi les halogènes et les groupes trifluorométhyle, nitro, cyano, alkyle en C₁-C₆, alcényle en C₂-C₆, alcynyle en C₂-C₆, alcoxy en C₁-C₆, alkylthio en C₁-C₆, alkylsulfonyle en C₁-C₆, hydroxy, hydroxyalkyle en C₁-C₆, amino, alkylamino en C₁-C₆, di(alkyl en C₁-C₆)amino, acyle, aminocarbonyle, (alkyl en C₁-C₆)aminocarbonyle et di(alkyl en C₁-C₆)aminocarbonyle; ou W est un groupe phénoxy ou phénylsulfanyle pouvant être substitué une ou plusieurs fois par des substituants choisis parmi les halogènes et les groupes trifluorométhyle, nitro, cyano, alkyle en C₁-C₆, alcényle en C₂-C₆, alcynyle en C₂-C₆, alcoxy en C₁-C₆, alkylthio en C₁-C₆, alkylsulfonyle en C₁-C₆, hydroxy, hydroxyalkyle en C₁-C₆, amino, alkylamino en C₁-C₆, di(alkyl en C₁-C₆)amino, acyle, aminocarbonyle, (alkyl en C₁-C₆)aminocarbonyle, di(alkyl en C₁-C₆)aminocarbonyle et éthylènedioxy et méthylènedioxy;
R¹, R², R³, R⁴, R¹¹, R¹² et R¹³ sont choisis parmi l'hydrogène, les halogènes et les groupes trifluorométhyle, nitro, cyano, alkyle en C₁-C₆, alcényle en C₂-C₆, alcynyle en C₂-C₆, alcoxy en C₁-C₆, alkylthio en C₁-C₆, alkylsulfonyle en C₁-C₆, hydroxy, hydroxyalkyle en C₁-C₆, amino, alkylamino en C₁-C₆, di(alkyl en C₁-C₆)amino, acyle, aminocarbonyle, (alkyl en C₁-C₆)aminocarbonyle et di(alkyl en C₁-C₆)aminocarbonyle;
n est 1 ou 2; m est 1, 2, 3, 4, 5 ou 6;
et ses sels d'addition d'acides.

2. Composé selon la revendication 1, dans lequel W est un groupe morpholino, pipéridinyle ou pyrrolyle éventuellement substitué.

3. Composé selon la revendication 2, dans lequel W est un groupe morpholino éventuellement substitué.

4. Composé selon la revendication 1, dans lequel W est un groupe phénoxy ou phénylsulfanyle éventuellement substitué.

5. Composé selon la revendication 1, dans lequel U est CR⁴, X est NR, Y est CH, Z est C et l'indole ainsi formé est lié au reste de la molécule par la position 3.

6. Composé selon les revendications 1-5, dans lequel V est N.

7. Composé selon les revendications 1 à 6, dans lequel m est 3, 4, 5 ou 6.

8. Composé selon la revendication 1, dans lequel Alk est un groupe méthyle.

9. Composition pharmaceutique, **caractérisée en ce qu'**elle comprend un composé selon l'une quelconque des revendications 1 à 8 avec un ou plusieurs supports ou diluants pharmaceutiquement acceptables.

10. Utilisation d'un composé selon l'une quelconque des revendications 1 à 8 pour la préparation d'un médicament utile dans le traitement de la psychose, des symptômes positifs et négatifs de la schizophrénie et des troubles affectifs comme l'anxiété généralisée, la panique, les troubles obsessionnels compulsifs, la dépression et l'agressivité.
